# EUROPEAN PATENT SPECIFICATION

(11) **EP 0 528 847 B1**
(45) Date of publication and mention of the grant of the patent: **28.09.1994**
(21) Application number: 91908609.0
(22) Date of filing: 19.04.1991
(51) Int. Cl.: C12N 15/12

(54) **SOLUBLE, TRUNCATED GAMMA-INTERFERON RECEPTORS**
LÖSLICHE VERKÜRZTE INTERFERON-GAMMA REZEPTOREN
RECEPTEURS DE GAMMA INTERFERON SOLUBLES TRONQUES

(30) Priority: 24.04.1990 US 513729
(43) Date of publication of application: 03.03.1993
(73) Proprietor: SCHERING CORPORATION, Kenilworth New Jersey 07033 (US)
(72) Inventor: ZAVODNY, Paul, J., Mountainside, NJ 07092 (US); NARULA, Satwant, K., West Caldwell, NJ 07006 (US); PETRO, Mary, E., Green Pond, NJ 07435 (US); LUNDELL, Daniel, J., Flemington, NJ 08822 (US); FOSSETTA, James, D., Roseland, NJ 08670 (US)
(74) Representative: Ritter, Stephen David
(86) International application number: US9102618
(87) International publication number: WO9116431

(56) References cited:
- EP-A- 0 240 975
- EP-A- 0 369 413
- EP-A- 0 393 502
- EP-A- 0 416 652
- J. of Interferon, suppl. 1, vol. 8, New York, US; D. Novick et al., p. S. 58
- The J. of Experimental Medicine, vol. 170, no. 4, 1 October 1989, D. Novick et al., pp. 1409-1414
- Cell, vol. 55, 21 Oct. 1988, M. Auguet et al., pp. 273-280
- J. of Chromatography, vol. 510, 27 June 1990, D. Novick et al., pp. 331-337
- Proceedings of the National Academy of Sciences, USA, vol. 85, July 1988, J. Calderon et al., pp. 4837-4841
- Immunology Today, vol. 9, no. 12, 1988, J.A. Langer et al., pp. 393-400

## Description

### TECHNICAL FIELD

This invention relates to soluble, truncated γ-interferon receptors, and in particular to soluble, extra-cellular portions of the γ-interferon receptor.

### BACKGROUND OF THE INVENTION

γ-Interferon, sometimes referred to herein as IFN-γ, is a cytokine produced by activated helper T cells; it has direct effects on several cell types such as B cells, macrophages and T cells. It has multiple effects (Trinchieri er al., Immunology Today, 6:131-136 (1985); one of its most distinguishing activities is that it induces expression of major histocompatibility complex (MHC) class I and class II genes (Kelley et al., J. Immunol., 132:240-245 (1984); Collins et al., Proc. Natl. Acad. Sci USA, 81:4917-4921 (1984); Cooper et al., J. Immunol., 141:1958-1962 (1988); and Amaldi et al., J Immunol., 142:999-1004 (1989)). The expression of MHC class II genes is a hallmark of antigen-presenting cells. Expression of class II antigens is seen in macrophages and mature B and T cells; and γ-interferon is known to upregulate this expression. In addition, γ-interferon is known to induce the expression of genes encoding class II antigens in cells that are not primary antigen-presenting cells, such as epithelial cells, fibroblasts, astrocytes, endothelial cells, and smooth muscle cells. Whether these types of cells do in fact present antigens is uncertain, but the induction of class II antigens in these cell types has been shown to correlate with the development of autoimmune disease (Massa et al., Proc. Natl. Acad. Sci. USA, 84:4219-4213 (1987)) . Since γ-interferon is the primary stimulator of class II antigens, it may play an important part in expression of the disease state. Therefore, an inhibitor of γ-interferon or its effects, for example one that acts at the receptor interface level, would have great potential value and utility in the treatment of auto-immune disease.

Attempts have been made to inhibit the effects of γ-interferon in mice by administering monoclonal antibodies against it (Grau et al., Proc. Natl. Acad. Sci. USA, 86:5572-5574 (1989)). However, in applying this treatment to humans, one would have to bear in mind the possibility that the monoclonal antibodies themselves may induce a neutralizing immune response that could reduce the effectiveness of this treatment.

Recombinant clones expressing γ-interferon receptors are known (see, for example, "Molecular Cloning and Expression of the Human Interferon-γ Receptor", Aguet, G., et al., Cell, 55:273-280 (1988)); but these provide the whole receptor, including the cytoplasmic (intracellular) domain and the trans-membrane domain. The cytoplasmic domain may be responsible for signal transduction in the cell, and is therefore superfluous when one is seeking an IFN-γ antagonist to seek out and selectively bind to IFN-γ in the blood stream or other body fluids (e.g. synovial fluid). Moreover, the cytoplasmic domain is normally concealed within the cell where it is inaccessible to the immune system; the full-length IFN-γ receptor could therefore conceivably act as an antigen by virtue of its cytoplasmic domain and be neutralized. If this happened, it could be removed and quickly lose its effect if introduced into such body fluids. There is thus a need for an antagonist to IFN-γ that competes for IFN-γ without the risk of inducing the side-effects associated with it.

The encoding DNA and predicted amino acid sequence of the IFN-γ receptor have been disclosed by Aguet et al. (supra), who have postulated that the leader sequence extends from amino acid (residue) 1 through amino acid 14, and that the transmembrane sequence extends from amino acid 246 through amino acid 266 (this is their numbering). The predicted extracellular portion of the IFN-γ receptor is therefore postulated to extend from amino acid 15 through amino acid 245; its postulated structure, including the leader sequence consisting of amino acid residues 1-14, and encoding DNA sequence are shown in SEQ ID NO 1, where the leader sequence bears the numbering -14 to -1, and the predicted extracellular portion bears the numbering 1 through 231.

In the following discussion, all the numbering will be given in accordance with that in SEQ ID NO 1, even though it may then vary from that in the publications referred to herein.

It is not certain how much of this sequence is necessary for an effective IFN-γ receptor that can specifically bind IFN-γ. However, it is known (Stueber et al., Abstract A3-15, J. Interferon Res., Vol. 9 Suppl. 2 (Oct. 1989)) that amino acid residues 1-198 do not provide an effective receptor whereas amino acid residues 12-231 (including sequences starting earlier than position 12) do provide an effective receptor as produced in *E. coli* according to that abstract, i.e., with six histidine residues at the N- or C-terminus. These histidine residues could conceivably change fundamental properties of the receptor, such as its immunogenicity, and are therefore highly undesirable when the soluble receptor is intended for use as a pharmaceutical. Our own work indicates that amino acid residues 6-221 do provide an effective receptor, but that other sequences could also be effective.

### SUMMARY OF THE INVENTION

The invention therefore provides a soluble receptor for γ-interferon, which consists of the glycosylated or unglycosylated extra-cellular moiety of the natural human γ-interferon receptor substantially free from other proteins, and has the formula given in SEQ ID NO 2;
wherein:
(Xaa)_{*n*} is the subsequence of amino-acids given in SEQ ID NO 5;
(Xaa)_{*p*} is a subsequence of one or more amino-acids starting from the amino terminus of the sequence given in SEQ ID NO 4;
m and n are both 1;
and p is 0 or 1.

This soluble receptor is thus part of the extracellular portion of the IFN-γ receptor, and it competes in a receptor-binding assay for IFN-γ and is an antagonist of the binding of IFN-γ to its cellular receptors; i.e., it competes with the IFN-γ receptors for IFN-γ. Furthermore, it can be used in a binding assay to measure IFN-γ: the procedure of Example 4D below can be adapted to a solid-phase format for a high through-put screen for IFN-γ antagonists.

The Ser and Arg residues at positions 1 and 2 are encoded by a potential ribosome binding site AGCAGG (usually AGGAGG; of. Shine and Dalgarno, Proc. Natl. Acad. Sci. USA 71:1342) (1974) (see SEQ ID NO 1), and there is an initiation codon 7-9 base pairs downstream. To eliminate the possibility of a false start in most prokaryotes, e.g. bacteria such as *E. coli*, we start at the Gly residue in position 6. In this way there may be provided an effective soluble IFN-γ receptor comprising amino acid residues 6-221, or in particular longer sequences, for example up to amino acid residue 231.

A full-length cDNA clone encoding the γ-interferon receptor (see, for example, Aguet et al., Cell, 55:273-280 (1988) (cited above)) was directly isolated by means of the polymerase chain reaction (Friedman et al., Nucl. Acids Res. 16:8718 (1988)) from a λgt11 placental cDNA library. The portion of the cDNA encoding the soluble portion of the IFN-γ receptor was isolated therefrom and incorporated into a plasmid that expresses it.

In one preferred embodiment of the invention, the soluble IFN-γ receptor is as defined above, wherein *p* is 0; i.e., the soluble IFN-γ receptor has the sequence of amino acids Ser+6-221; *i.e.*, the sequence of SEQ ID NO: 2 wherein *m* is 1, (Xaa)_{*n*} is as defined in SEQ ID NO: 5, and *p* is 0.

In another particularly preferred embodiment of the invention, the soluble IFN-γ receptor is as defined above, wherein *p* is 1, and the sequence represented by (Xaa)_{*p*} is completely present; i.e., the soluble IFN-γ receptor has the sequence of amino acids Ser+6-231; *i.e.*, the sequence of SEQ ID NO: 2 wherein *m* is 1, (Xaa)_{*n*} is as defined in SEQ ID NO: 5, *p* is 0, and (Xaa)_{*p*} is as defined in SEQ ID NO: 4.

The invention further provides a pharmaceutical composition comprising an effective amount of the soluble receptor of the invention and a pharmaceutically acceptable carrier or excipient.

The invention further provides a method for inhibiting the binding of IFN-γ to cells having receptors for IFN-γ, which comprises the steps of:
administering an effective amount of the soluble IFN-γ receptor of the invention to a medium containing cells having receptors for IFN-γ; and
allowing said soluble receptor to compete for the cell receptors.

### BRIEF DESCRIPTION OF THE FIGURES:

Figure 1 shows the plasmid pDSRS into which the DNA sequence encoding the soluble IFN-γ receptor was inserted.

Figure 2 shows schematically how an *E. coli* expression plasmid for the soluble IFN-γ receptor was constructed.

Figure 3 depicts the competition of the soluble IFN-γ receptor protein produced in *E. coli* with IFN-γ binding on U937 cells; the binding assays were carried out as described in Example 4D.

### DESCRIPTION OF THE INVENTION

All references cited herein are incorporated in their entirety by reference.

Expression vectors encoding and capable of producing the extracellular domain of the IFN-γ receptor cDNA in prokaryotes were used for obtaining prokaryotic clones that produced the soluble (extracellular) portion of the IFN-γ receptor. Prokaryotic cells that can be used include bacteria, especially *E. coli*; many strains of *E. coli* can be used in practising the present invention. However, the preferred *E. coli* strain is a leaky mutant that allows the soluble IFN-γ receptor to escape from the periplasmic space into the culture medium, whence it can be readily isolated without the complications caused by the presence of most other *E. coli* proteins. The production of such bacteria and their use in the manufacture of recombinant heterologous proteins is disclosed in copending application no. 07/429,588, filed October 31, 1989, which is hereby incorporated by reference.

Several different *E. coli* promoters, such as Trp, lac, lpp, λpL, etc., can be used in the practice of the invention. These promoters direct the expression of heterologous proteins in the cytoplasm. For effective secretion into the periplasmic space, a signal peptide sequence such as one derived from *E. coli* OmpA, *E. coli* alkaline phosphatase, *E. coli* lipoprotein, etc., may be used. The expression plasmid for prokaryotic expression in *E. coli* contains a strong promoter, especially a tandem double promoter such as lpp-lac, followed by a DNA sequence encoding a signal sequence that can transport the heterologous protein into the periplasmic space. This last DNA sequence is preferably the signal sequence derived from an *E. coli* outer-membrane protein, OmpA. The signal sequence is followed by the coding sequence of the IFN-γ receptor cDNA.

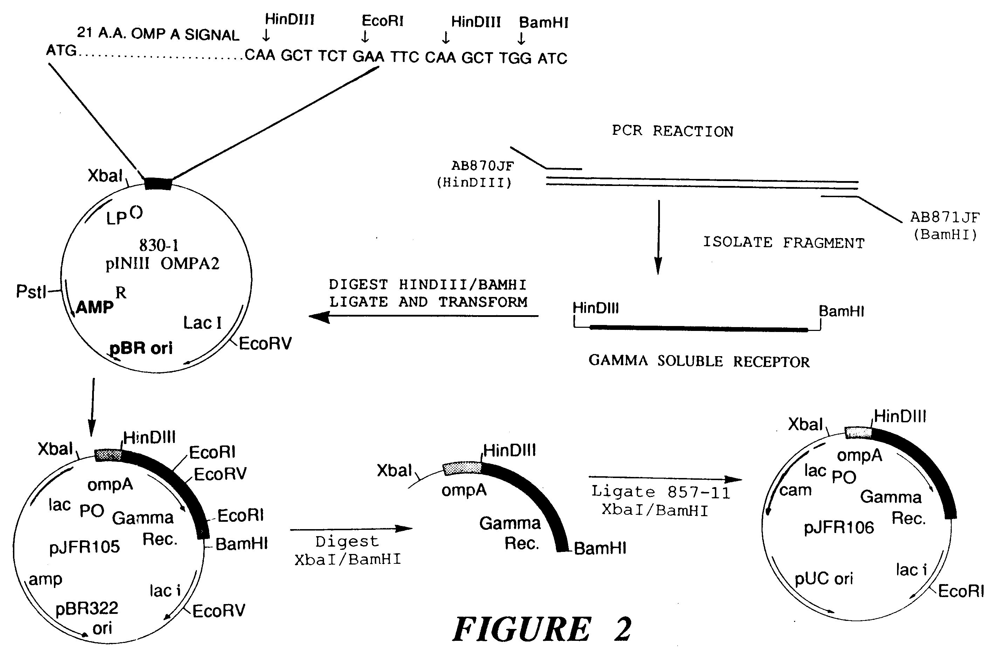

The construction scheme (shown in Figure 2) used in making this plasmid introduced one additional residue, *viz* serine, at the start of the mature coding sequence. A translation STOP codon was introduced at the end of the sequence encoding the predicted soluble IFN-γ receptor. A plasmid containing these attributes, when used to transform a mutant *E. coli* strain (*e.g.*, the strain available from the ATCC under Accession No. 53956), produces a fusion protein that is composed of the OmpA signal sequence followed by the sequence of the soluble IFN-γ receptor. The fusion protein is further processed in the periplasm to yield the mature soluble IFN-γ receptor, which leaks into the culture medium.

Eukaryotic cell-lines that can be used include mammalian cell-lines, e.g. COS7, NS-1 and CHO cells, and yeast cells; additionally, insect expression systems, *e.g. Bombyx mori* or *Spodoptera frugiperda*, can be used.

To demonstrate this invention, U937 cells were used as a convenient source of γ-interferon receptors; however, the soluble receptors described herein will of course inhibit the binding of IFN-γ to any cells that bear those receptors. Such cells include, for example, B cells, T cells, eosinophiles, smooth muscle cells, promyelocytes, macrophages, erythroid cells, monocytes, granulocytes, etc.

### PURIFICATION

Example 3 below describes the purification of the soluble IFN-γ receptor from *E. coli*, but its procedure can be adapted to the purification of the receptor from other sources such as eukaryotic cells, especially mammalian cells and yeast cells.

### MATERIALS AND METHODS

1. REAGENTS
   Restriction enzymes were purchased from New England Biolabs., Beverly, MA. *Thermus aquaticus* DNA polymerase and 10X buffer were purchased from Stratagene, LaJolla, CA. Double-stranded plasmid DNA sequencing was done using Sequenase Version 2.0 from United States Biochemical, Cleveland, OH. A λgt11 human placental cDNA library was purchased from Clontech, Palo Alto, CA.
2. SYNTHETIC OLIGONUCLEOTIDES
   The following synthetic oligonucleotides were synthesized by standard methods with an Applied Biosystems DNA Synthesizer Model 380A:
   AB697: See SEQ ID NO 6;
   AB758: See SEQ ID NO 7;
   AB759: See SEQ ID NO 8;
   AB812: See SEQ ID NO 9;
   AB813: See SEQ ID NO 10;
   AB870JF: See SEQ ID NO 11;
   AB871JF: See SEQ ID NO 12.
3. The polymerase chain reaction (PCR) was carried out as previously described (Friedmann et al., Nucleic Acids Res. (1988), 16:8718).
4. Human IFN-γ can be purified according to the procedure of Example 4A or be obtained commercially, and antibodies thereto can also be obtained commercially. For example, Genzyme Corp. (Boston, MA) supplies recombinant human IFN-γ (99% pure) under the code HG-IFN, and also rabbit polyclonal anti-human IFN-γ under the code IP-500 for Western blotting.

### EXAMPLES

### EXAMPLE 1. ISOLATION AND TRANSIENT EXPRESSION OF THE FULL-LENGTH IFN-γ RECEPTOR CLONE

The oligonucleotides AB758 and 759, given in SEQ ID NOS 7 and 8, which are identical to the 5'- and 3'-untranslated regions of the IFN-γ receptor cDNA sequence, were used in a PCR with placental cDNA library phage lysate essentially as previously described (Friedman et al., Nucl. Acids Res. (1988), 16:8718). The PCR was run with a Techne programmable Dri-Block (GRI, Essex, UK), under the following conditions: denaturation at 95°C for 2 minutes, primer annealing at 50°C for 2 minutes, and chain extension at 72°C for 2 minutes, all for 30 cycles, with a final cycle elongation at 72°C for 9 minutes. The PCR products were electrophoresed in a 1% agarose gel, and a main band of approximately 1.5 kb corresponding to the full-length IFN-γ receptor coding region was visualized by staining with ethidium bromide and isolated by electroelution. The authenticity of the fragment was confirmed with a second PCR using the oligomer AB758, given in SEQ ID NO 7, as a 5'-primer and AB697, given in SEQ ID NO 6, as an internal primer corresponding to sequences in the intracellular region of the IFN-γ receptor, to produce a fragment of approximately 1.2 kb.

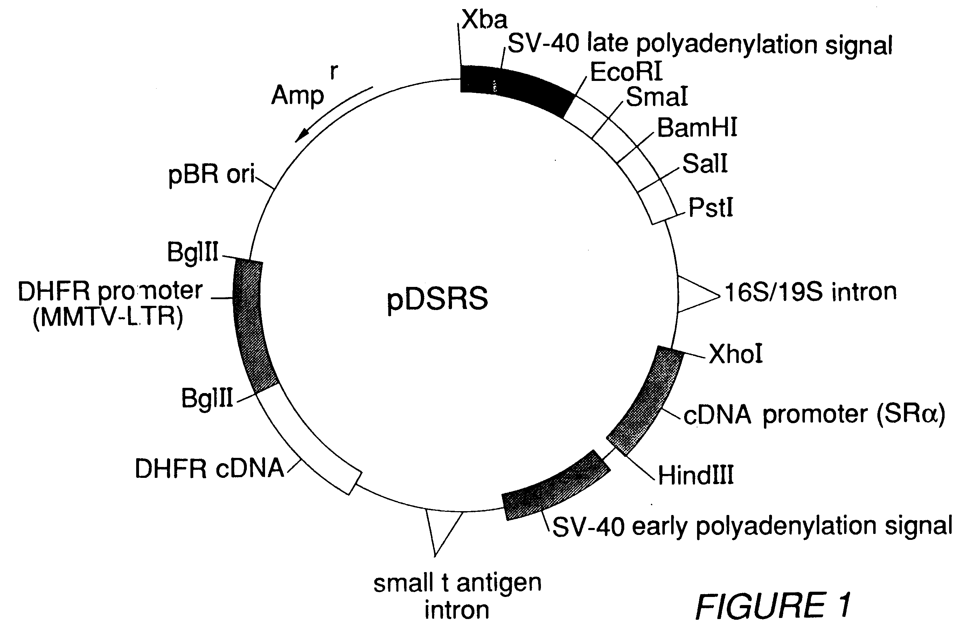

Restriction sites for PstI (5') and SalI (3') were introduced onto the 1.5 kb full-length IFN-γ receptor fragment with oligomers AB812 and AB813, given in SEQ ID NOS 9 and 10, in a PCR as described above. The resulting fragment was isolated, digested with PstI and SalI and ligated into PstI/SalI-digested pDSRS plasmid (ATCC Accession No. 68232) (Fig. 1), for expression in COS7 cells. The resulting ligation mixture was used to transform competent *E. coli* 294 (readily available from E. coli Genetics Stock Center, Dept. of Biology, Yale University, P. O. Box 6666, New Haven, CT 06511-7444).

Plasmid DNA isolated by the alkaline lysis method (Birnboim and Doly, "A rapid alkaline extraction procedure for screening recombinant plasmid DNA", Nucl. Acids Res., 7:1513 (1978)) from fourteen of the resulting clones was analysed and checked by restriction analysis and PCR. Seven clones contained the 1.5 kb IFN-γ receptor fragment. DNA from six of these seven clones was purified by cesium chloride/ethidium bromide gradient centrifugation (Maniatis et al.: "Molecular cloning: A Laboratory Manual", Cold Spring Harbor Laboratory, Cold Spring Harbor, NY) and was transfected transiently into COS7 cells (5 µg DNA/100mM dish) by the DEAE-dextran method essentially as described (McCutchan and Pagano, "Enhancement of the infectivity of Simian Virus 40 Deoxyribonucleic Acid with Diethylamino-ethyl-Dextran", J. Natl. Cancer Inst., 41:351-356 (1968)). After growth at 37°C for 60-72 hours, the cells were harvested by mild trypsinization and resuspended to 1 x 10⁷ cells/ml in RPMI medium containing 0.02% sodium azide, and kept at 4°C. They were then checked for expression of the IFN-γ receptor by the specific binding of ¹²⁵I-labeled IFN-γ, using techniques described in Example 4 below.

Three clones showed a marked increase in specific binding of ¹²⁵I-labeled γ-interferon (see Table I below). Plasmid DNA from all three clones was sequenced by the dideoxy chain-termination method (Sanger et al., "DNA Sequencing with Chain-terminating Inhibitors", Proc. Natl. Acad. Sci. U.S.A., 74, 5463-6567 (1977)); the sequence of the extracellular domain was identical to the published sequence (Aguet et al., *supra*).

### EXAMPLE 2. CONSTRUCTION OF pJFR105-6 FOR EXPRESSION OF GENE ENCODING SOLUBLE IFN-γ RECEPTOR IN E. COLI

Using a DNA fragment isolated from a cDNA library and coding for the γ-interferon receptor, primers were made to introduce two sites for cloning the soluble domain into a prokaryotic expression plasmid. Primer AB870JF, given in SEQ ID NO 12, was made to introduce a HindIII restriction site close to the amino terminus of the DNA (which would be the *Gly* in position 6); and primer AB871JF, given in SEQ ID NO 13, was made to introduce a BamHI restriction site and a STOP codon, which would serve to isolate and define only the soluble domain of the molecule. (These primers isolate and define the soluble domain of the IFN-γ receptor minus its own leader sequence; i.e., the polypeptide sequence between the leader sequence and the transmembrane region.) Using PCR technology, the DNA fragment (encoding the full-length receptor) and primers were combined in a reaction mixture as follows: 100 pmoles primers, 25 ng DNA, 10 mM dNTPs, 10 µl TAQ polymerase buffer, and 2 units TAQ polymerase. The system was set at 30 cycles for annealing, denaturation and synthesis, in a total reaction volume of 100 µl. After the PCR, the DNA was separated on a 1% agarose gel, and the 800 bp fragment encoding the γ-interferon receptor was visualized by staining with ethidium bromide and isolated from the agarose.

Plasmid p830-1 (pINIII, OmpA leader) (D. Lundell et al., "Cytoplasmic and Periplasmic Expression of a Highly Basic Protein, Human Interleukin 4, in *E. coli*", J. Indust. Microbiol., vol. 4 (1989), and available on request from R. Kastelein at DNAX Research Institute of Molecular and Cellular Biology, Inc., 901 California Avenue, Palo Alto, CA 94304-1104) contains HindIII and BamHI restriction sites, which were used to clone the IFN-γ receptor fragment in frame downstream of the OmpA leader as follows: 20 µg of plasmid DNA was digested with 50 units of HindIII restriction nuclease and then with 40 units of BamHI restriction nuclease. After digestion, the mixture of restriction fragments was run out on a 0.65% agarose gel; a 7.5 kb fragment representing the plasmid backbone was isolated. Any other appropriate plasmid encoding a suitable secretory signal could have been used instead.

The 7.5 kb fragment (0.1 µg) representing the plasmid backbone and 1 µg of the 800 kb fragment encoding the γ-interferon receptor were ligated in a mixture containing ATP, T4 DNA ligase (100 units), and dithiothreitol at 16°C and in a volume of 40 µl for 14 hours. Half of the product was then used to transform CaCl₂-prepared *E. coli* (K12) 294 (readily available from E. coli Genetics Stock Center, Dept. of Biology, Yale University, P. O. Box 6666, New Haven, CT 06511-7444). The mixture from the transformation was plated onto ampicillin plates and incubated at 30°C for 16-24 hours. Several colonies were then picked and fermented, and DNA was isolated and analysed by digestion with the enzymes mentioned above (HindIII, BamHI), and the clone pJFR105-6 was selected.

The pJFR105-6 clone has the fragment inserted downstream of OmpA and under the control of the lpp/lac promoter. The plasmid is derived from pBR322 and carries the LAC-I gene so that expression is inducible with isopropyl-thio-β-galactoside (IPTG).

### EXAMPLE 3. PURIFICATION OF SOLUBLE HUMAN γ-INTERFERON RECEPTOR FROM E. COLI

The extracellular domain of the human IFN-γ receptor was purified from *E. coli* harboring plasmid pJFR105-6. Cells were grown at 30°C in M9 - casamino acids medium [1 x M9 minimal salts (Gibco BRL No. M29800B), 3% (w/v) casamino acids, 2 g/l glucose, 0.1 g/l thiamine, and 0.2 g/l magnesium sulfate] to A₆₆₀ = 1.0. IPTG was added to 0.5 mM, and fermentation was continued at 30°C overnight (final culture A₆₆₀ = 3.5) . Conditioned media from a 12-liter culture were collected and then clarified by centrifugation. The clarified supernatant was adjusted to 5% (w/v) trichloroacetic acid (TCA), and protein was allowed to precipitate at 4°C for 60 minutes. The insoluble fraction was collected by centrifugation at 10,000 x g, and resuspended to 120 ml in 200 mM Tris (pH 8) for resolubilization. After incubation at 4°C for 60 minutes, the residue from the resolubilized fraction was collected by centrifugation. The supernatant was adjusted to 4 M urea, 20 mM Tris (pH 8), incubated at 4°C for 30 minutes and then applied to a DEAE Sephadex® Fast Flow column (Pharmacia No. 17-0709-01), equilibrated with a buffer of 20 mM Tris (pH 8), 4 M urea. The column was then eluted with a linear 0 - 0.3 M sodium chloride gradient in the same Tris/urea buffer. The soluble extracellular domain of the human IFN-γ receptor eluted at approximately 0.18 M NaCl. The soluble receptor pool was dialysed against 20 mM Tris (pH 8), and applied to a 5 ml IFN-γ-Affigel 10 column, equilibrated with 20 mM Tris. [Human IFN-γ was covalently coupled to Affigel 10 (Biorad Catalog No. 153-6046), according to procedures recommended by the manufacturer. This affinity resin contained 4 mg IFN-γ/ml support resin.] The column was washed with 10 ml 0.2 M NaCl in 200 mM Tris (pH 8), and then with 10 ml 0.5 M NaCl in 200 mM Tris (pH 8). The soluble receptor was then eluted with 200 mM sodium carbonate buffer, pH 10.3, 0.6 M NaCl. The fractions were neutralized and pooled, and then dialysed against 20 mM sodium phosphate, pH 7.5.

This procedure yielded from 0.7 to 1 mg of soluble IFN-γ receptor per 12-liter fermentation. However, about 50% of the soluble IFN-γ receptor was present in the TCA-insoluble fraction. This material could be denatured and refolded to yield further active protein.

### EXAMPLE 4. ASSAY FOR SOLUBLE γ-INTERFERON RECEPTORS

### A. Purification of Human γ-Interferon

*E. coli* 294 harboring plasmid pGIF4-137 (a pBR322-based expression vector having the bacterial lipoprotein (lpp) promoter and encoding a human γ-interferon gene (amino acids 4-137 of the mature protein)) was grown in modified GC medium (20 g/l glycerol, 30 g/l casamino acids (Difco), 20 g/l yeast extract (Difco), 5 g/l KH₂PO₄, and 1 g/l MgSO₄.7H₂O) at 37°C. At A₆₆₀ = 7-10, cells were harvested and lysed by sonic disruption. A cell-free insoluble fraction was then isolated by centrifugation, resuspended in 20 mM Tris (pH 8), 6 M guanidine hydrochloride and 5 mM dithiothreitol, and resolubilized by heating at 56°C for 30 min.

This resolubilized fraction was then loaded onto a 3 x 90 cm Sepracyl 200-SF® column (Pharmacia AB), equilibrated in 20 mM Tris (pH 8), 6 mM guanidine hydrochloride at room temperature. After fractionation the fractions containing the γ-interferon (*e.g.*, as detected by Western blot analysis) were pooled, diluted 120-fold in 10 mM. NH₄OAc (pH 7), and mixed at 4°C overnight. SP-Sephadex® resin (Sigma Chemical Co.) (10 ml as a 1:1 slurry in 20 mM Tris, pH 8) was added to this sample and it was mixed at 4°C for 60 minutes. The resin was filtered off and washed with 10 mM NH₄OAc (pH 7), and purified γ-interferon was eluted with 1 mM NaCl. The purity of this γ-interferon fraction was greater than 95%, as shown by SDS polyacrylamide gel electrophoresis.

### B. Labeling of Human γ-Interferon

¹²⁵I-labeled human γ-interferon was prepared using Bolton Hunter reagent (New England Nuclear) according to procedures described by the supplier (the labeling reaction contained 70 µg/ml γ-interferon in 20 mM sodium phosphate at pH 8). The final reaction mixture was desalted by dialysis and applied to a 1 x 24 cm Sephadex G75® column equilibrated in PBS and 0.2% gelatin to separate polymerized γ-interferon from dimer protein. The γ-interferon pool, labeled to 50 Ci/mM, was stored at 4°C until use.

### C. Sample Preparation Using E. coli

*E. coli* 732I (ATCC Accession No. 53956), transformed with the plasmid pJFR105-6, was grown in nutrient medium at 37°C to A₆₆₀ = 1. The culture was then induced with 0.1 mM IPTG, and growth was continued at 37°C for 3 hours. Supernatants from induced cultures were checked for expression of the soluble IFN-γ receptor using the binding assay described below.

### D. Binding Assay

U-937 cells (ATCC Catalog No. CRL 1593) were inoculated from 5% dimethyl sulfoxide, 95% fetal bovine serum frozen stocks into prewarmed RPMI 1640 medium (Hazelton Biologics, Inc.) and then grown and passaged at 37°C to cell densities not exceeding 1 x 10⁶ cells/ml. The cells were collected by centrifugation immediately before use, resuspended to 1.25 x 10⁷ cells/ml in RPMI 1640 medium containing 0.02% sodium azide, and stored or ice. All subsequent procedures were carried out at 4°C.

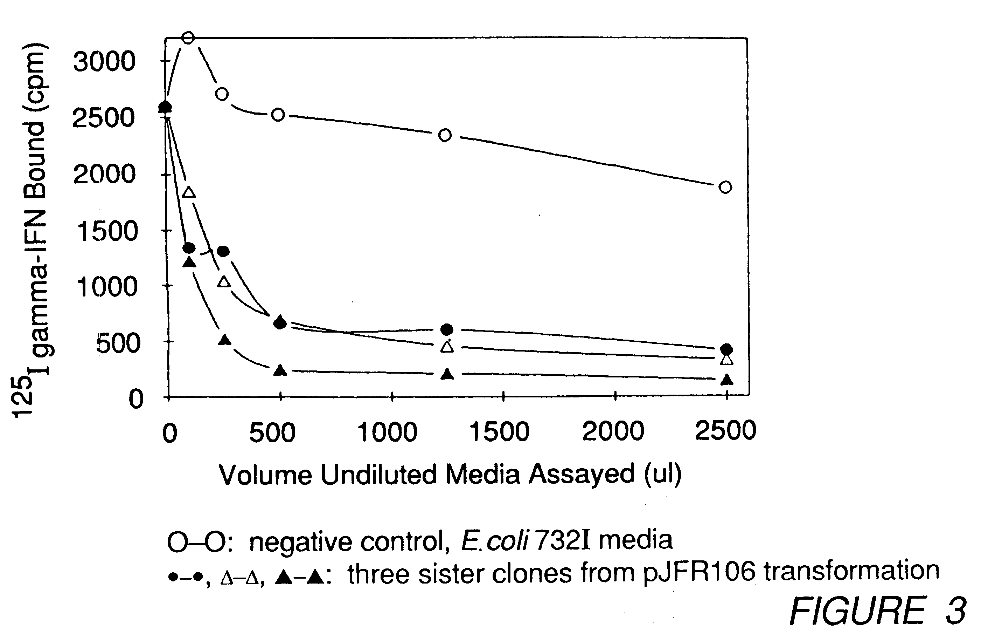

Receptor binding assays were performed as follows:
1.25 x 10⁶ U937 cells were used per assay. (Each assay provided a point in Fig. 3.) The ¹²⁵I-IFN-γ was added at 10,000 cpm equivalents. Supernatant from the culture of cells transfected with plasmid T892-5B (expressing the gene for the soluble receptor) was added at increasing volumes ranging from 0 to 100 ml. Three components were present in the assay: U-937 cells, ¹²⁵I-labelled IFN-γ, and sample containing soluble IFN-γ receptor. Mixtures containing two of these three components were incubated at 4°C for 30 minutes. The third component was then added and incubation was continued at 4°C for 120 minutes. The cells were centrifuged through oil [150 µl of a 1:1 mixture of dioctyl phthalate (Aldrich Chemical Co.) and dibutyl phthalate (Eastman Kodak Co.)]. The tubes were then quick-frozen, cell pellets were excised from the bottom of each tube, and radioactivity associated with the pellets was determined in a γ-counter.

By way of example, one way of carrying out the foregoing procedure is as follows:
Dilutions of conditioned media suspected of containing soluble receptor were added (in 50 µl RPMI media) into wells of a 96-well microtiter plate. Labeled γ-interferon (approximately 50,000 cpm in 50 µl) was added to each well, followed by 100 µl of the U-937 cell suspension (1.25 x 10⁷ cells/ml). After incubation at 4°C for 2 hours, each reaction mixture was pipetted into a 0.4 ml micro test-tube (Bio-Rad) containing 150 µl dioctyl phthalate:dibutyl phthalate (1:1). The assay tubes were centrifuged in a swinging bucket rotor and frozen in liquid nitrogen, and the tube tip (containing the frozen cell pellet separated from the reaction liquor by the oil layer) was excised and analysed for radioactivity in a gamma counter. Media fractions able to inhibit the binding of γ-interferon to the U-937 cells were identified as containing soluble IFN-γ receptor.

The results are shown in Table I.

**TABLE I**

| SCREEN FOR EXPRESSION OF CLONED FULL-LENGTH γ-INTERFERON RECEPTOR IN COS7 CELLS | | | | |
|---|---|---|---|---|
| Expt. No. | Plasmid No. | Background Binding (1) | Background Binding (2) | Specific Binding (3) |
| 1 | T884-5 | 1832.8 | 129.8 | 1703.0 |
| 1' | T884-5 | 1560.5 | 77.8 | 1482.7 |
| 2 | T886-5 | 1985.0 | 150.4 | 1834.6 |
| 2' | T886-5 | 2404.6 | 197.6 | 2207.0 |
| 3 | T886-6 | 2525.9 | 230.9 | 2295.0 |
| 3' | T886-6 | 2092.8 | 69.8 | 2023.0 |
| 4 | No Insert | 110.7 | 136.8 | 0 |
| 4' | No Insert | 159.4 | 160.0 | 0 |
| 5 | No DNA | 125.4 | 184.4 | 0 |
| 5' | No DNA and no chloroquine | 158.1 | 178.3 | 0 |

| | | | | |
|---|---|---|---|---|
| (1) ¹²⁵I IFN-γ cpm bound at 0.1 µg/ml unlabeled IFN-γ. | | | | |
| (2) ¹²⁵I IFN-γ cpm bound at 10 µg/ml unlabeled IFN-γ. | | | | |
| (3) Specific binding = (1) - (2). | | | | |

Figure 3 shows the results of experiments in which the binding of the complete γ-interferon receptor on U937 cells with γ-interferon is compared with the binding of the soluble γ-interferon receptor of this invention with γ-interferon. In this receptor binding assay, the soluble γ-interferon receptor, produced in *E. coli*, competed in a dose-dependent manner in preventing the binding of ¹²⁵I-labelled γ-interferon to its receptors on U937 cells.

These data show that the soluble receptor is a competitive inhibitor for the binding of γ-interferon to its cellular receptors. In laboratory test procedures (*e.g.*, screening assays) using this competitive inhibition, the IFN-γ or its soluble receptor can be tagged with a label that is able to generate a signal, e.g., a radiolabel or a chemical label, especially a fluorescent label. Moreover, by virtue of this competitive inhibition, the soluble γ-interferon receptor of the present invention is also likely to find therapeutic use in the treatment of auto-immune diseases such as rheumatoid arthritis, multiple sclerosis, Sjögren's Syndrome and lupus erythematosus.

The soluble IFN-γ receptor of the invention can be administered as a pharmaceutical composition. Such compositions contain a therapeutically effective amount of the soluble receptor of the invention and a pharmaceutical carrier or excipient. A pharmaceutical carrier can be any compatible, non-toxic substance suitable for delivering the soluble IFN-γ receptor of the invention to a patient. Sterile water, alcohol, fats, waxes, and inert solids may for example be included in a carrier, and pharmaceutically acceptable adjuvants (*e.g.*, buffering agents, dispersing agents) may also be incorporated. Generally, compositions useful for parenteral administration of such drugs are well known, e.g. see Remington's Pharmaceutical Sciences, 14th Ed. (Mack Publishing Company, Easton, PA 1980). Alternatively, compositions of the invention may be introduced into a patient's body by an implantable drug delivery system; e.g. see Urquhart et al., Ann. Rev. Pharmacol. Toxicol, vol. 24, pgs. 199-236 (1984).

The soluble IFN-γ receptor of the invention is normally administered parenterally, preferably intravenously. Although the soluble IFN-γ receptor is not expected to be immunogenic, it is preferably administered slowly, either by a conventional IV administration set or from a subcutaneous depot.

When administered parenterally, the soluble IFN-γ receptor will normally be formulated with a pharmaceutically acceptable parenteral vehicle in a unit dosage form suitable for injection (e.g., a solution, suspension or emulsion). Such vehicles are inherently non-toxic and non-therapeutic. Examples of such vehicles are normal saline, Ringer's solution, dextrose solution, and Hank's solution. Non-aqueous vehicles such as fixed oils and ethyl oleate may also be used. A preferred vehicle is 5% dextrose/saline. The vehicle may contain minor amounts of additives such as substances that enhance isotonicity and chemical stability, e.g., buffers and preservatives. The soluble IFN-γ receptor is preferably formulated in purified form substantially free of aggregates, degradation products and contaminating proteins, at concentrations of about 5 to 500 µg/ml, preferably 20 to 250 µg/ml.

Selecting an administration regimen for the soluble IFN-γ receptor depends on several factors, including the serum turnover rate of the soluble IFN-γ receptor, the serum level of IFN-γ associated with the auto-immune disorder, any possible immunogenicity of the soluble IFN-γ receptor, the accessibility of the target IFN-γ, the affinity of IFN-γ to its cellular receptor(s) relative to that of IFN-γ to the soluble IFN-γ receptor, and the like.

Determination of the proper dosage of a compound of the invention for a particular situation is within the skill of the art. Usually, treatment is initiated with dosages that are less than the optimum dose. Thereafter, the dosage is increased by small amounts until the optimum effect under the circumstances is reached. For convenience, the total daily dosage may be divided and administered in portions during the day if desired.

The amount and frequency of administration of the soluble IFN-γ receptor will be regulated according to the judgment of the attending clinician considering such factors as age, condition and size of the patient as well as severity of the symptoms being treated.

According to the administration regimen, the amount of soluble IFN-γ receptor delivered to the patient is preferably maximized consistent with an acceptable level of side effects; and the amount delivered therefore depends in part on the severity of the disease being treated. Preferably, the dose is in the range of abort 0.1 to 500 µg/kg per day, more preferably about 1 to 50 µg/kg per day.

A typical recommended dosage regimen is parenteral administration of from 1 µg/day to 5 mg/day, preferably 20 µg/day to 1 mg/day, in two to four divided doses to achieve relief of the auto-immune symptoms.

The descriptions of the foregoing embodiments of the invention have been presented for purpose of illustration and description. They are not intended to be exhaustive or to limit the invention to the precise forms disclosed, and obviously many modifications and variations are possible in the light of the above teachings. The embodiments were chosen and described to explain the principles of the invention and its practical application, so that others skilled in the art would thereby be enabled to use and practise appropriately such embodiments and modifications of the invention as are suited to the particular use contemplated. It is intended that the scope of the invention be defined by the claims appended hereto.

SEQUENCE LISTING

SEQ ID NO: 1

SEQUENCE TYPE: DNA Sequence with corresponding amino acid
sequence

SEQUENCE LENGTH: 735 bases; 245 amino acid residues

STRANDEDNESS: single

TOPOLOGY: linear

MOLECULE TYPE: DNA molecule and encoded protein/poly-
peptide

ORIGINAL SOURCE ORGANISM: Human

PROPERTIES: Soluble receptor for γ-interferon

FEATURES:
from 1 to 42: DNA encoding leader sequence
from 43 to 735: DNA encoding human mature soluble
gamma-Interferon receptor

ATG GCT CTC CTC TTT CTC CTA CCC CTT GTC ATG CAG GGT GTG 42
Met Ala Leu Leu Phe Leu Leu Pro Leu Val Met Gln Gly Val
-10 -5

AGC AGG GCT GAG ATG GGC ACC GCG GAT CTG GGG CCG TCC TCA GTG 87
Ser Arg Ala Glu Met Gly Thr Ala Asp Leu Gly Pro Ser Ser Val
1               5                   10                  15

CCT ACA CCA ACT AAT GTT ACA ATT GAA TCC TAT AAC ATG AAC CCT 132
Pro Thr Pro Thr Asn Val Thr Ile Glu Ser Tyr Asn Met Asn Pro
                20                  25                  30

ATC GTA TAT TGG GAG TAC CAG ATC ATG CCA CAG GTC CCT GTT TTT 177
Ile Val Tyr Trp Glu Tyr Gln Ile Met Pro Gln Val Pro Val Phe
                35                  40                  45

ACC GTA GAG GTA AAG AAC TAT GGT GTT AAG AAT TCA GAA TGG ATT 222
Thr Val Glu Val Lys Asn Tyr Gly Val Lys Asn Ser Glu Trp Ile
                50                  55                  60

GAT GCC TGC ATC AAT ATT TCT CAT CAT TAT TGT AAT ATT TCT GAT 267
Asp Ala Cys Ile Asn Ile Ser His His Tyr Cys Asn Ile Ser Asp
                65                  70                  75

CAT GTT GGT GAT CCA TCA AAT TCT CTT TGG GTC AGA GTT AAA GCC 312
His Val Gly Asp Pro Ser Asn Ser Leu Trp Val Arg Val Lys Ala
                80                  85                  90

AGG GTT GGA CAA AAA GAA TCT GCC TAT GCA AAG TCA GAA GAA TTT 357
Arg Val Gly Gln Lys Glu Ser Ala Tyr Ala Lys Ser Glu Glu Phe
                95                  100                 105

GCT GTA TGC CGA GAT GGA AAA ATT GGA CCA CCT AAA CTG GAT ATC 402
Ala Val Cys Arg Asp Gly Lys Ile Gly Pro Pro Lys Leu Asp Ile
                110                 115                 120

AGA AAG GAG GAG AAG CAA ATC ATG ATT GAC ATA TTT CAC CCT TCA 447
Arg Lys Glu Glu Lys Gln Ile Met Ile Asp Ile Phe His Pro Ser
                125                 130                 135

GTT TTT GTA AAT GGA GAC GAG CAG GAA GTC GAT TAT GAT CCC GAA 492
Val Phe Val Asn Gly Asp Glu Gln Glu Val Asp Tyr Asp Pro Glu
                140                 145                 150

ACT ACC TGT TAC ATT AGG GTG TAC AAT GTG TAT GTG AGA ATG AAC 537
Thr Thr Cys Tyr Ile Arg Val Tyr Asn Val Tyr Val Arg Met Asn
                155                 160                 165

GGA AGT GAG ATC CAG TAT AAA ATA CTC ACG CAG AAG GAA GAT GAT 582
Gly Ser Glu Ile Gln Tyr Lys Ile Leu Thr Gln Lys Glu Asp Asp
                170                 175                 180

TGT GAC GAG ATT CAG TGC CAG TTA GCG ATT CCA GTA TCC TCA CTG 627
Cys Asp Glu Ile Gln Cys Gln Leu Ala Ile Pro Val Ser Ser Leu
                185                 190                 195

AAT TCT CAG TAC TGT GTT TCA GCA GAA GGA GTC TTA CAT GTG TGG 672
Asn Ser Gln Tyr Cys Val Ser Ala Glu Gly Val Leu His Val Trp
                200                 205                 210

GGT GTT ACA ACT GAA AAG TCA AAA GAA GTT TGT ATT ACC ATT TTC 717
Gly Val Thr Thr Glu Lys Ser Lys Glu Val Cys Ile Thr Ile Phe
                215                 220                 225

AAT AGC AGT ATA AAA GGT. 735
Asn Ser Ser Ile Lys Gly.
230

SEQ ID NO: 2
SEQUENCE TYPE: Amino acid sequence
SEQUENCE LENGTH: 231 amino acid residues
STRANDEDNESS: single
TOPOLOGY: linear
MOLECULE TYPE: protein/polypeptide
ORIGINAL SOURCE ORGANISM: Human
PROPERTIES: Soluble receptor for γ-interferon
(no leader sequence)
For (Xaa)n and (Xaa)p see SEQ ID NOS 4 and 5 respectively

(Ser)m (Xaa)n Ser Ser Val Pro Thr Pro Thr Asn Val Thr Ile Glu
1 13 15 20

Ser Tyr Asn Met Asn Pro Ile Val Tyr Trp Glu Tyr Gln Ile Met
25 30 35

Pro Gln Val Pro Val Phe Thr Val Glu Val Lys Asn Tyr Gly Val
40 45 50

Lys Asn Ser Glu Trp Ile Asp Ala Cys Ile Asn Ile Ser His His
55 60 65

Tyr Cys Asn Ile Ser Asp His Val Gly Asp Pro Ser Asn Ser Leu
70 75 80

Trp Val Arg Val Lys Ala Arg Val Gly Gln Lys Glu Ser Ala Tyr
85 90 95

Ala Lys Ser Glu Glu Phe Ala Val Cys Arg Asp Gly Lys Ile Gly
100 105 110

Pro Pro Lys Leu Asp Ile Arg Lys Glu Glu Lys Gln Ile Met Ile
115 120 125

Asp Ile Phe His Pro Ser Val Phe Val Asn Gly Asp Glu Gln Glu
130 135 140

Val Asp Tyr Asp Pro Glu Thr Thr Cys Tyr Ile Arg Val Tyr Asn
145 150 155

Val Tyr Val Arg Met Asn Gly Ser Glu Ile Gln Tyr Lys Ile Leu
160 165 170

Thr Gln Lys Glu Asp Asp Cys Asp Glu Ile Gln Cys Gln Leu Ala
175 180 185

Ile Pro Val Ser Ser Leu Asn Ser Gln Tyr Cys Val Ser Ala Glu
190 195 200

Gly Val Leu His Val Trp Gly Val Thr Thr Glu Lys Ser Lys Glu
205 210 215

Val Cys (Xaa)p
220

SEQ ID NO: 3

SEQUENCE TYPE: Amino acid sequence represented by (Xaa)n
in SEQ. ID. NO. 2

SEQUENCE LENGTH: 11 amino acid residues

STRANDEDNESS: single

TOPOLOGY: linear

MOLECULE TYPE: polypeptide

ORIGINAL SOURCE ORGANISM: Human

PROPERTIES: amino acid residues nos. 2 to 12 of soluble
receptor for γ-interferon

Arg Ala Glu Met Gly Thr Ala Asp Leu Gly Pro
                5                   10

SEQ ID NO: 4

SEQUENCE TYPE: Amino acid sequence represented by (Xaa)p
in SEQ. ID. NO. 2

SEQUENCE LENGTH: 10 amino acid residues

STRANDEDNESS: single

TOPOLOGY: linear

MOLECULE TYPE: polypeptide

ORIGINAL SOURCE ORGANISM: Human

PROPERTIES: amino acid residues nos. 222 to 231 of soluble
receptor for γ-interferon

Ile Thr Ile Phe Asn Ser Ser Ile Lys Gly
225 230

SEQ ID NO: 5

SEQUENCE TYPE: Sub-sequence of amino acid sequence
represented by (Xaa)n in SEQ. ID. NO. 2

SEQUENCE LENGTH: 7 amino acid residues

STRANDEDNESS: single

TOPOLOGY: linear

MOLECULE TYPE: polypeptide

ORIGINAL SOURCE ORGANISM: Human

PROPERTIES: amino acid residues nos. 6 to 12 of soluble
receptor for γ-interferon

Gly Thr Ala Asp Leu Gly Pro
 6              10

SEQ ID NO: 6

SEQUENCE TYPE: Synthetic DNA sequence designated AB697

SEQUENCE LENGTH: 23 bases

STRANDEDNESS: single

TOPOLOGY: linear

MOLECULE TYPE: DNA sequence

ORIGINAL SOURCE ORGANISM: None

PROPERTIES: internal primer corresponding to the
complement of the sequence in the intracellular region of
the IFN-γ receptor

CAGACTGGTT ACTACTTAAA GGT                                23

SEQ ID NO: 7

SEQUENCE TYPE: Synthetic DNA sequence designated AB758

SEQUENCE LENGTH: 20 bases

STRANDEDNESS: single

TOPOLOGY: linear

MOLECULE TYPE: DNA sequence

ORIGINAL SOURCE ORGANISM: None

PROPERTIES: 5'-primer corresponding to sequence in the
5'-untranslated region of the IFN-γ receptor cDNA sequence

CAGCGACCGT CGGTAGCAGC 20

SEQ ID NO: 8

SEQUENCE TYPE: Synthetic DNA sequence designated AB759

SEQUENCE LENGTH: 20 bases

STRANDEDNESS: single

TOPOLOGY: linear

MOLECULE TYPE: DNA sequence

ORIGINAL SOURCE ORGANISM: None

PROPERTIES: 3'-primer corresponding to the complement of
the sequence in the 3'-untranslated region of the IFN-γ
receptor cDNA

CTTCAAAGTT GGTGCAACTT 20

SEQ ID NO: 9

SEQUENCE TYPE: Synthetic DNA sequence designated AB812

SEQUENCE LENGTH: 27 bases

STRANDEDNESS: single

TOPOLOGY: linear

MOLECULE TYPE: DNA sequence

ORIGINAL SOURCE ORGANISM: None

PROPERTIES: oligomer to introduce PstI restriction site
into the end of the 1.5 kb full-length IFN-γ receptor
fragment

CTATCTGCAG CGACCGTCGG TAGCAGC 27

SEQ ID NO: 10

SEQUENCE TYPE: Synthetic DNA sequence designated AB813

SEQUENCE LENGTH: 30 bases

STRANDEDNESS: single

TOPOLOGY: linear

MOLECULE TYPE: DNA sequence

ORIGINAL SOURCE ORGANISM: None

PROPERTIES: oligomer to introduce SalI restriction site
into the 3'-end of the 1.5 kb full-length IFN-γ receptor
fragment

GTATGTCGAC TTCCAAAGTT GGTGCAACTT 30

SEQ ID NO: 11

SEQUENCE TYPE: Synthetic DNA sequence designated AB870JF

SEQUENCE LENGTH: 39 bases

STRANDEDNESS: single

TOPOLOGY: linear

MOLECULE TYPE: DNA sequence

ORIGINAL SOURCE ORGANISM: None

PROPERTIES: 5'-primer to introduce a HindIII restriction
site close to the codon for glycine (in position no. 6) at
the amino terminus of the DNA encoding the soluble IFN-γ
receptor

GCGCAAGCTT CTGGCACCGC GGATCTGGGG CCGTCCTCA 39

SEQ ID NO: 12

SEQUENCE TYPE: Synthetic DNA sequence designated AB871JF

SEQUENCE LENGTH: 39 bases

STRANDEDNESS: single

TOPOLOGY: linear

MOLECULE TYPE: DNA sequence

ORIGINAL SOURCE ORGANISM: None

PROPERTIES: 3'-primer to introduce a BamHI restriction
site and a STOP codon at the extra-cellular/transmembrane
junction of the IFN-γ receptor (i.e., the STOP codon after
glycine at position 231)

GGCGGATCCT TAACCTTTTA TACTGCTATT GAAAATGAA 39

## Claims (Claims for the following Contracting State(s): AT, BE, CH, DE, DK, FR, GB, GR, IT, LI, LU, NL, SE)

1. A soluble receptor for γ-interferon (IFN-γ), which consists of the glycosylated or unglycosylated extra-cellular moiety of the natural human IFN-γ transmembrane cellular receptor substantially free from other proteins, and has the formula given in SEQ ID NO 2;
wherein:
(Xaa)_{*n*} is the subsequence of amino-acids given in SEQ ID NO 5;
(Xaa)_{*p*} is a subsequence of one or more amino-acids starting from the amino terminus of the sequence given in SEQ ID NO 4;
m and n are both 1;
and p is 0 or 1.

2. The soluble receptor of claim 1 wherein *p* is 0; i.e., the soluble IFN-γ receptor has the sequence of amino acids Ser+6-221; *i.e.*, the sequence of SEQ ID NO: 2 wherein *m* is 1, (Xaa)_{*n*} is as defined in SEQ ID NO: 5, and *p* is 0.

3. The unglycosylated soluble receptor of claim 1.

4. The soluble receptor of claim 1 wherein *p* is 1, and the sequence represented by (Xaa)_{*p*} is completely present; i.e., the soluble IFN-γ receptor has the sequence of amino acids Ser+6-231; *i.e.*, the sequence of SEQ ID NO: 2 wherein *m* is 1, (Xaa)_{*n*} is as defined in SEQ ID NO: 5, *p* is 0, and (Xaa)_{*p*} is as defined in SEQ ID NO: 4.

5. A pharmaceutical composition comprising an effective amount of the soluble receptor of claim 1 and a pharmaceutically acceptable carrier or excipient.

6. A pharmaceutical composition comprising an effective amount of the soluble receptor of claim 3 and a pharmaceutically acceptable carrier or excipient.

7. A method for inhibiting the binding of IFN-γ to cells having receptors for IFN-γ, which comprises the steps of:
administering an effective amount of the soluble IFN-γ receptor of claim 1 to a medium containing cells having receptors for IFN-γ; and
allowing said soluble receptor to compete for the cell receptors.

8. A method for inhibiting the binding of IFN-γ to cells having receptors for IFN-γ, which comprises the steps of:
administering an effective amount of the soluble IFN-γ receptor of claim 4 to a medium containing cells having receptors for IFN-γ; and
allowing said soluble receptor to compete for the cell receptors.

## Claims (Claims for the following Contracting State(s): ES)

1. A method of manufacturing a soluble receptor for γ-interferon (IFN-γ), which consists of the glycosylated or unglycosylated extra-cellular moiety of the natural human IFN-γ transmembrane cellular receptor substantially free from other proteins, comprising transforming a suitable cell such as *E.coli*, COS7, NS-1 or CH0 with a plasmid capable of inducing expression of a polypeptide having the amino acid sequences given in SEQ ID NO 2;
wherein
(Xaa)*n* is the subsequence of amino-acids given in SEQ ID NO 5;
(Xaa)*p* is a subsequence of one or more amino-acids starting from the amino terminus of the sequence given in SEQ ID NO 4;
m and n are both 1;
and p is 0 or 1,
growing a culture of the cells and extracting the soluble receptor therefrom.

2. A method according to Claim 1 wherein *p* is 0; i.e., the soluble IFN-γ receptor has the sequence of amino acids SER+6-221; i.e., the sequence of SEQ ID NO: 2 wherein *m* is 1, (Xaa)*n* is as defined in SEQ ID NO:5, and *p* is 0.

3. A method according to Claim 1 wherein the receptor is unglycosylated.

4. A method according to Claim 1 wherein *p* is 1, and the sequence represented by (Xaa)*p* is completely present; i.e., the soluble IFN-γ receptor has the sequence of amino acids Ser+6-231; i.e., the sequence of SEQ ID NO: 2 wherein *m* is 1, (Xaa)*n* is as defined in SEQ ID NO: 5, *p* is 0, and (Xaa)*p* is as defined in SEQ ID NO:4.

5. A method of manufacturing a pharmaceutical composition comprising mixing an effective amount of the soluble receptor of any of Claims 1-4 with a pharmaceutically acceptable carrier or excipient.

## Patentansprüche (Patentansprüche für folgende(n) Vertragsstaat(en): AT, BE, CH, DE, DK, FR, GB, GR, IT, LI, LU, NL, SE)

1. Löslicher Rezeptor für γ-Interferon (IFN-γ), der aus der glykosylierten oder unglykosylierten, extrazellulären Struktureinheit des transmembranen Zellrezeptors des natürlichen Human-IFN-γ besteht, im wesentlichen frei von anderen Proteinen ist und die in SEQ ID Nr. 2 angegebene Formel besitzt, worin:
(Xaa)ₙ die Untersequenz der in SEQ ID Nr. 5 angegebenen Aminosäuren ist,
(Xaa)ₚ eine Untersequenz einer oder mehrerer Aminosäuren vom Aminoende aus der in SEQ ID Nr. 4 angegebenen Sequenz ist,
m und n beide 1 sind
und p 0 oder 1 ist.

2. Löslicher Rezeptor von Anspruch 1, bei welchem p 0 ist, d.h. der lösliche IFN-γ-Rezeptor besitzt die Sequenz der Aminosäuren Ser+6-221, d.h. die Sequenz von SEQ ID Nr. 2, bei welcher m 1 ist, (Xaa)ₙ wie in SEQ ID Nr. 5 definiert ist und p 0 ist.

3. Unglykosylierter, löslicher Rezeptor des Anspruchs 1.

4. Löslicher Rezeptor des Anspruchs 1, bei welchem p 1 ist und die durch (Xaa)ₚ dargestellte Sequenz vollständig vorhanden ist, d.h. der lösliche IFN-γ-Rezeptor besitzt die Sequenz der Aminosäuren Ser+6-231, d.h. die Sequenz von SEQ ID Nr. 2, bei welcher m 1 ist, (Xaa)ₙ wie in SEQ ID Nr. 5 definiert ist, p 0 ist und (Xaa)ₚ wie in SEQ ID Nr. 4 definiert ist.

5. Pharmazeutische Zusammensetzung, die eine wirksame Menge des löslichen Rezeptors des Anspruchs 1 und einen pharmazeutisch annehmbaren Träger oder Verdünnungsmittel umfaßt.

6. Pharmazeutische Zusammensetzung, die eine wirksame Menge des löslichen Rezeptors des Anspruchs 3 und einen pharmazeutisch annehmbaren Träger oder Verdünnungsmittel umfaßt.

7. Verfahren zum Hemmen des Bindens von IFN-γ an Zellen mit Rezeptoren für IFN-γ, welches die Schritte
des Verabreichens einer wirksamen Menge des löslichen IFN-γ-Rezeptors des Anspruchs 1 an ein Medium, das Zellen mit Rezeptoren für IFN-γ enthält, und
Konkurrierenlassen des löslichen Rezeptors um die Zellrezeptoren umfaßt.

8. Verfahren zum Hemmen des Bindens von IFN-γ an Zellen mit Rezeptoren für IFN-γ, welches die Schritte
des Verabreichens einer wirksamen Menge des löslichen IFN-γ-Rezeptors des Anspruchs 4 an ein Medium, das Zellen mit Rezeptoren für IFN-γ enthält, und
Konkurrierenlassen des löslichen Rezeptors um die Zellrezeptoren umfaßt.

## Patentansprüche (Patentansprüche für folgende(n) Vertragsstaat(en): ES)

1. Verfahren zum Herstellen eines löslichen Rezeptors für γ-Interferon (IFN-γ), der aus der glykosylierten oder unglykosylierten, extrazellulären Struktureinheit des transmembranen Zellrezeptors des natürlichen Human-IFN-γ besteht, im wesentlichen frei von anderen Proteinen ist, welches das Transformieren einer geeigneten Zelle wie etwa *E. coli*, COS7, NS-1 oder CHO mit einem Plasmid, das zum Auslösen der Expression eines Polypeptids mit den in SEQ ID Nr. 2 angegebenen Aminosäuresequenzen, worin:
(Xaa)ₙ die Untersequenz der in SEQ ID Nr. 5 angegebenen Aminosäuren ist,
(Xaa)ₚ eine Untersequenz einer oder mehrerer Aminosäuren vom Aminoende aus der in SEQ ID Nr. 4 angegebenen Sequenz ist,
m und n beide 1 sind
und p 0 oder 1 ist,
und das Züchten einer Kulter der Zellen und Extrahieren des löslichen Rezeptors daraus umfaßt.

2. Verfahren gemäß Anspruch 1, bei welchem p 0 ist, d.h. der lösliche IFN-γ-Rezeptor besitzt die Sequenz der Aminosäuren Ser+6-221, d.h. die Sequenz von SEQ ID Nr. 2, bei welcher m 1 ist, (Xaa)ₙ wie in SEQ ID Nr. 5 definiert ist und p 0 ist.

3. Verfahren gemäß Anspruch 1, bei welchem der Rezeptor unglykosyliert ist.

4. Verfahren gemäß Anspruch 1, bei welchem p 1 ist und die durch (Xaa)ₚ dargestellte Sequenz vollständig vorhanden ist, d.h. der lösliche IFN-γ-Rezeptor besitzt die Sequenz der Aminosäuren Ser+6-231, d.h. die Sequenz von SEQ ID Nr. 2, bei welcher m 1 ist, (Xaa)ₙ wie in SEQ ID Nr. 5 definiert ist, p 0 ist und (Xaa)ₚ wie in SEQ ID Nr. 4 definiert ist.

5. Verfahren zum Herstellen einer pharmazeutischen Zusammensetzung, welches das Mischen einer wirksamen Menge des löslichen Rezeptors eines der Ansprüche 1-4 mit einem pharmazeutisch annehmbaren Träger oder Verdünnungsmittel umfaßt.

## Revendications (Revendications pour l'(les) Etat(s) contractant(s) suivant(s): AT, BE, CH, DE, DK, FR, GB, GR, IT, LI, LU, NL, SE)

1. Récepteur soluble pour interféron-γ (IFN-γ), qui consiste d'une moitié extra-cellulaire glycosylatée ou non-glycosylatée du récepteur cellulaire de transmembrane IFN-γ humain naturel essentiellement exempt d'autres protéines, et qui a la formule donnée dans SEQ ID N°2;
où :
(Xaa)_{*n*} est la suite des acides aminés données en SEQ ID N°5;
(Xaa)_{*p*} est une suite de un ou plusieurs acides aminés démarrant du terminus amino de la séquence donnée en SEQ ID N°4;
m et n sont tous les deux 1;
et p est 0 ou 1.

2. Récepteur soluble selon la revendication 1, dans lequel *p* est 0; c'est-à-dire, que le récepteur soluble IFN-γ a la séquence d'acides aminés Ser+6-221; c'est-à-dire, la séquence de SEQ ID N°:2 où *m* est 1, (Xaa)_{*n*} est tel que défini dans SEQ ID N°:5, et *p* est 0.

3. Récepteur soluble non glycosylaté de la revendication 1.

4. Récepteur soluble de la revendication 1 où *p* est 1, et la séquence représentée par (Xaa)*p* est complètement présente; c'est-à-dire que, le récepteur soluble IFN-γ a la séquence des acides aminés Ser+6-231; c'est-à-dire, la séquence de SEQ ID N°:2 où *m* est 1, (Xaa)*n* est comme défini dans SEQ ID N°:5, *p* est 0, et (Xaa)*p* est tel que défini en SEQ ID N°:4.

5. Composition pharmaceutique comprenant une quantité efficace du récepteur soluble de la revendication 1 et un porteur ou excipient pharmaceutiquement acceptable.

6. Composition pharmaceutique comprenant une quantité efficace du récepteur soluble de la revendication 3, et un excipient ou porteur pharmaceutiquement acceptable.

7. Méthode pour inhiber la liaison de IFN-γ aux cellules ayant des récepteurs pour IFN-γ, qui comprend les étapes de :
administrer une quantité efficace du récepteur soluble IFN-γ de la revendication 1 à un milieu contenant des cellules ayant des récepteurs pour IFN-γ; et
laisser ledit récepteur soluble être en compétition pour les récepteurs de cellules.

8. Méthode pour inhiber la liaison de IFN-γ aux cellules ayant des récepteurs pour IFN-γ, qui comprend les étapes de :
administrer une quantité efficace du récepteur soluble IFN-γ de la revendication 4 à un milieu contenant des cellules ayant des récepteurs pour IFN-γ; et
laisser ledit récepteur soluble entrer en compétition pour les récepteurs de cellules.

## Revendications (Revendications pour l'(les) Etat(s) contractant(s) suivant(s): ES)

1. Méthode de fabrication d'un récepteur soluble pour l'interféron-γ (IFN-γ), qui consiste de la moitié extra-cellulaire glycosylatée ou non glycosylatée du récepteur cellulaire transmembrane IFN-γ humain naturel essentiellement exempt d'autres protéines, comprenant la transformation d'une cellule appropriée telle que *E.coli*, COS7, NS-1 ou CHO avec un plasmide capable d'induire l'expression d'un polypeptide ayant les séquences acides aminés données en SEQ ID N°2;
où
(Xaa)*n* est la suite des acides aminés données en SEQ ID N°5;
(Xaa)*p* est une suite d'un ou plusieurs acides aminés démarrant du terminus amino de la séquence donnée dans SEQ ID N°4;
m et n sont tous les deux 1;
et p est 0 ou 1,
la croissance d'une culture des cellules et l'extraction du récepteur soluble de celle-ci.

2. Méthode selon la revendication 1 où *p* est 0; c'est-à-dire que, le récepteur soluble IFN-γ a la séquence des acides aminés SER+6-221, c'est-à-dire, la séquence SEQ ID N°:2 où *m* est 1, (Xaa)_{*n*} est tel que défini dans SEQ ID N°:5, et *p* est 0.

3. Méthode selon la revendication 1 où le récepteur est non glycosylaté.

4. Méthode selon la revendication 1, où *p* est 1, et la séquence représentée par (Xaa)*p* est complètement présente; c'est-à-dire que le récepteur soluble IFN-γ a la séquence des acides aminés Ser+6-231; c'est-à-dire la séquence de SEQ ID N°:2 où *m* est 1, (Xaa)*n* est tel que défini dans SEQ ID N°:5, *p* est 0, et (Xaa)*p* est tel que défini dans SEQ ID N°:4.

5. Méthode de fabrication d'une composition pharmaceutique comprenant le mélange d'une quantité efficace du récepteur soluble selon l'une quelconque des revendications 1 à 4, avec un excipient ou porteur pharmaceutiquement acceptable.
